(19) Europäisches Patentamt
European Patent Office
Office européen des brevets

(11) EP 3 209 758 B1

(12) # EUROPEAN PATENT SPECIFICATION

(45) Date of publication and mention of the grant of the patent:
**15.08.2018 Bulletin 2018/33**

(21) Application number: **15787841.4**

(22) Date of filing: **21.10.2015**

(51) Int Cl.:
***C11D 1/83*** *(2006.01)* ***C11D 3/20*** *(2006.01)*
***C11D 3/30*** *(2006.01)*

(86) International application number:
**PCT/US2015/056608**

(87) International publication number:
**WO 2016/064968 (28.04.2016 Gazette 2016/17)**

(54) **LAUNDRY DETERGENT CONTAINING AMINE ADDITIVES**

WASCHMITTEL MIT AMINADDITIVEN

DÉTERGENT POUR LESSIVE CONTENANT DES ADDITIFS D'AMINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.10.2014 EP 14290320**

(43) Date of publication of application:
**30.08.2017 Bulletin 2017/35**

(73) Proprietor: **Rohm and Haas Company**
**Philadelphia, PA 19106 (US)**

(72) Inventor: **DELLA NOCE, Gaelle**
**06560 Valbonne (FR)**

(74) Representative: **Houghton, Mark Phillip**
**Patent Outsourcing Limited**
**Cornerhouse**
**1 King Street**
**Bakewell**
**Derbyshire DE45 1DZ (GB)**

(56) References cited:
**WO-A1-00/20545**
**WO-A1-2012/067962**
**WO-A1-2012/161979**
**WO-A2-2009/125335**
**US-A- 4 079 078**
**US-A- 5 035 838**
**US-A1- 2011 230 380**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 209 758 B1

## Description

Background

This invention relates generally to a concentrated laundry detergent composition containing amines.

Typically, liquid laundry detergent compositions are adjusted to an alkaline pH using sodium hydroxide. In some cases, other bases have been used for this purpose. For example, U.S. Pat. No. 5,035,838 discloses use of triethanolamine for neutralization of a detergent composition. Documents US 4,079,078, WO 2009/125335 A2 and WO 2012/067962 disclose liquid detergent compositions neutralized with various amines. However, these references do not disclose the compositions of the present invention, which offer improved performance.

Statement of Invention

The present invention is directed to a laundry detergent composition comprising: (a) 4 to 25 wt% of a linear alkyl benzene sulfonate; (b) 25 to 55 wt% nonionic surfactant, (c) 2 to 10 wt% of an amine having formula (I)

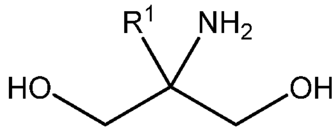

(I)

wherein $R^1$ is methyl or ethyl; or formula (II)

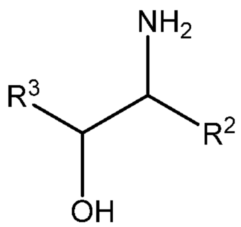

(II)

wherein $R^2$ is $C_1$-$C_4$ alkyl and $R^3$ is $C_2$-$C_8$ alkyl; or a combination thereof; and (d) 10 to 35 wt% $C_3$-$C_6$ polyhydroxy compounds.

Detailed Description

All percentages are weight percentages (wt%), and all temperatures are in °C, unless otherwise indicated. All operations are performed at room temperature (20-25 °C) unless otherwise specified. As used herein the term "citrate" refers to alkali metal citrates. Preferably, citrates are sodium, potassium or lithium salts; preferably sodium or potassium; preferably sodium. As used herein the term "fatty acid" refers to aliphatic carboxylic acids having from twelve to twenty-two carbon atoms. Weight percentages of citrates are based on the actual weights of the salts, including metal ions. The term "phosphorus-free" refers to compositions containing less than 0.5 wt% phosphorus (as elemental phosphorus), preferably less than 0.2 wt%, preferably less than 0.1 wt%, preferably no detectable phosphorus. Weight percentages in the detergent composition are percentages of commercial products, i.e., including any water that may be present in the products. $R^1$ is methyl or ethyl.

Preferably, $R^2$ is $C_1$-$C_3$ alkyl, preferably methyl or ethyl, preferably ethyl. Preferably, $R^3$ is $C_2$-$C_6$ alkyl, preferably $C_3$-$C_6$ alkyl, preferably $C_3$-$C_5$ alkyl, preferably $C_4$ alkyl, preferably n-butyl.

Preferably, the total amount of amine in the detergent composition is from 2.5 to 9 wt%, preferably from 2.5 to 7 wt%, preferably from 3 to 5 wt%, preferably from 3 to 4 wt%.

Preferably, the detergent composition comprises at least 30 wt% nonionic surfactants, preferably at least 32 wt%, preferably at least 34 wt%; preferably no more than 50 wt%, preferably no more than 47 wt%, preferably no more than 44 wt%, preferably no more than 42 wt%. Preferably, the nonionic surfactant is a linear alcohol ethoxylate. Preferably, a linear alcohol ethoxylate has a linear $C_6$-$C_{16}$ alkyl group, preferably $C_8$-$C_{14}$. Preferably the alkyl groups are mixtures derived from seed oil, preferably comprising 70% $C_8$-$C_{10}$ linear alkyl and 70% $C_{12}$-$C_{14}$ linear alkyl. Preferably, a linear alcohol ethoxylate contains from five to nine polymerized units of ethylene oxide, preferably seven. Preferably, a linear alcohol ethoxylate has two to four polymerized units of propylene oxide between the alkyl group and the ethylene oxide

units, preferably three units of propylene oxide.

Preferably, the detergent composition comprises at least 6 wt% linear alkylbenzene sulfonates, preferably at least 8 wt%, preferably at least 10 wt%; preferably no more than 20 wt%, preferably no more than 18 wt%, preferably no more than 16 wt%, preferably no more than 14 wt%. Preferably, alkylbenzene sulfonates have a $C_{10}$-$C_{14}$ alkyl group.

Preferably, the detergent composition comprises from 10 to 35 wt% $C_3$-$C_5$ polyhydroxy compounds, preferably 15 to 30 wt%, preferably 20 to 30 wt%. Polyhydroxy compounds preferably have two or three hydroxyl groups. Preferred polyhydroxy compounds include, e.g., glycerol and propylene glycol. Preferably, the detergent composition comprises from 5 to 25 wt% propylene glycol, preferably 8 to 20 wt%, preferably 10 to 18 wt%.

Preferably, the detergent composition comprises from 1 to 15 wt% fatty acids or their metal salts; preferably at least 2 wt%, preferably at least 3 wt %; preferably no more than 10 wt%, preferably no more than 8 wt%, preferably no more than 6 wt%.

Preferably, the detergent composition comprises from 10 to 30 wt% alkyl ether sulfate, preferably from 12 to 25 wt%, preferably from 14 to 23 wt%. Preferably, an alkyl ether sulfate has a $C_8$-$C_{18}$ alkyl group, preferably $C_{10}$-$C_{16}$. Preferably, the alkyl group is linear.

Preferably, the detergent composition comprises no more than 20 wt% water, preferably no more than 16 wt%, preferably no more than 14 wt%; preferably at least 2 wt% water, preferably at least 4 wt%, preferably at least 6 wt%, preferably at least 8 wt%.

Other components of the detergent composition may include, e.g., surfactants, oxygen and/or chlorine bleaches, bleach activators, enzymes, foam suppressants, colors, fragrances, antibacterial agents and fillers. Fillers in tablets or powders are inert, water-soluble substances, typically sodium or potassium salts, e.g., sodium or potassium sulfate and/or chloride, and typically are present in amounts ranging from 0 wt% to 75 wt%. Fillers in gel formulations may include those mentioned above and also water. Fragrances, dyes, foam suppressants, enzymes and antibacterial agents usually total no more than 5 wt% of the composition.

Preferably, the composition has a pH from 7.5 to 10, preferably at least 8, preferably no more than 9.5, preferably no more than 9.

The composition can be formulated in any typical form, e.g., as a monodose, liquid or gel. The composition can be used under typical operating conditions for any typical washing machine. Typical water temperatures during the washing process preferably are from 20°C to 85°C, preferably from 25°C to 70°C. Typical concentrations for the composition as a percentage of total liquid in the washing machine preferably are from 0.1 to 1 wt%, preferably from 0.2 to 0.7 wt%. With selection of an appropriate product form and addition time, the composition may be present in the prewash, main wash, penultimate rinse, final rinse, or any combination of these cycles.

Preferably, the detergent composition is substantially free of enzymes, i.e., it contains less than 0.5 wt% enzymes, preferably less than 0.2 wt%, preferably less than 0.1 wt%, preferably it contains no detectable enzymes.

Preferably, the detergent composition contains less than 2 wt% of amine oxide surfactants, preferably less than 1 wt%, preferably less than 0.5 wt%, preferably less than 0.2 wt%. Preferably, the detergent composition is substantially free of amines other than the amines of formulas (I) and (II), i.e., these other amines are present in a total amount less than 0.7 wt%, preferably less than 0.5 wt%, preferably less than 0.3 wt%, preferably less than 0.2 wt%.

Examples

The composition of the DOW monodose formulation is given below:

| ***Dow monodose composition (in order of addition)*** | ***% as product*** |
|---|---|
| Propylene glycol (hydrotrope) | 14.22 |
| Sodium Lauryl Ether Sulfate (STEOL CS 370E from Stepan) | 18.96[1] |
| Alcohol ethoxylate[3] | 37.91 |
| Fatty Acid (PALMERA B1220 form KLK) | 4.27 |
| Glycerin (PURENE from DOW) | 9.48 |
| Neutralizer from Angus | 3.32 |

(continued)

| ***Dow monodose composition (in order of addition)*** | ***% as product*** |
|---|---|
| Linear Alkyl Benzene Sulfonate (NANSA SS50 from Huntsman) | 11.85[2] |
| 1. This product contains 30% water.<br>2. This product contains 50% water; total water in formulation: 11.6%<br>3. Alkyl groups are mixtures comprising 70% $C_8$-$C_{10}$ linear alkyl and 70% $C_{12}$-$C_{14}$ linear alkyl with seven polymerized units of ethylene oxide and three polymerized units of propylene oxide between the alkyl group and the ethylene oxide units. | |

The following amines have been tested in this monodose formulation:

- MEA (comp.) (mono-ethanolamine)
- Tris Amino Ultra PC (2-amino-2-hydroxymethyl-1.3-propanediol)
- CORRGUARD EXT (3-amino-4-octanol)
- AMP 90 (2-amino-2-methyl-1-propanol)
- AEPD Vox 1000 (2-amino-2-ethyl-1,3-propanediol)
- AMPD Ultra PC (2-amino-2-methyl-1,3-propanediol)

The stain monitors used for the test were as follow:

***Stain monitors: from Center for Test Materials- Ref MON DOW 7***

| ***Code stains*** | ***Nature of stains*** |
|---|---|
| H078 | Lipstick #1 |
| H156 | Dust sebum |
| H038 | Shoepolish brown |
| H150 | Soja oil / violet dye |
| H013 | Dirty motor oil |
| H032 | Rust stain |
| H084 | nivea visage face cream |
| H135 | Mechanical Grease |
| H082 | Sebum bey with carbon black |
| H066 | tapenada, black olives |
| H018 | Ground soil |
| H134 | Hamburger grease |
| H126 | Pura vegetable oil with violet dye |
| H056 | Sauce Mediterranea, olive sauce |
| H162 | Chicken barbecue fat |
| H145 | Clay tenniscourt |

Each machine was loaded with:

✓ 1 stain monitor Mon Dow 7 + 1 SBL (soil ballast load of 8g)
✓ 33 g detergent
✓ 3.3kg ballast load
✓ Tap water: TH around 18°F
✓ Wash temperature 40°C

**Measurements**

The soil release effect has been measured via Delta E (ΔE) of each stain.

Delta E is defined as the color difference between the unwashed stain and the washed stain, within the L*a*b* color space.

Each stain was measured, before and after washing, with the Epson Perfection 4490 Photo scanner, using the Epson scan software with the following settings:

- Professional mode.
- Document type: reflective
- Documents source: Document table
- Auto exposure type: photo
- Image type: 48-bit color
- scanner quality : best
- Resolution: 400dpi
- No color correction
- UV cut 400 nm

Then, the color of each stain have been analyzed with the software Image J version 1.4.3.67 (free software available from the Internet) using a macro which provided color information within the L*a*b* color space.

***Results (based on 1 cycle at 40°C)***

Delta E is defined as the color difference between the unwashed stain and the washed stain, within the L*a*b* color space.

$$\Delta E = \sqrt{((L*_{unwashed} - L*_{washed})^2 + (a*_{unwashed} - a*_{washed})^2 + (b*_{unwashed} - b*_{washed})^2)}$$

The Delta E of each stain is summarized in the table below:

| Stain | MEA | Tris Amino Ultra PC | CORRGUARD EXT | AMP 90 | AEPD Vox 1000 | AMPD Ultra PC |
|---|---|---|---|---|---|---|
| H078 | 26.05 | 32.28 | 26.51 | 28.58 | 42.72 | 39.95 |
| H156 | 13.98 | 12.79 | 14.33 | 14.74 | 12.58 | 13.72 |
| H038 | 55.20 | 47.71 | 53.38 | 50.68 | 45.38 | 56.81 |
| H150 | 16.82 | 18.34 | 21.76 | 20.82 | 20.95 | 24.42 |
| H013 | 15.05 | 14.60 | 17.89 | 15.35 | 17.23 | 14.33 |
| H032 | 5.01 | 4.29 | 7.40 | 5.29 | 5.87 | 4.35 |
| H084 | 27.70 | 29.02 | 38.33 | 35.26 | 36.14 | 30.06 |
| H135 | 8.50 | 7.02 | 12.23 | 8.92 | 8.51 | 9.45 |
| H082 | 50.42 | 51.04 | 47.03 | 47.32 | 51.11 | 48.98 |
| H066 | 35.35 | 37.51 | 36.00 | 35.25 | 35.71 | 39.39 |
| H018 | 29.02 | 30.95 | 35.17 | 37.74 | 32.59 | 31.56 |
| H134 | 5.84 | 5.16 | 6.70 | 5.63 | 5.29 | 5.88 |

(continued)

| Stain | MEA | Tris Amino Ultra PC | CORRGUARD EXT | AMP 90 | AEPD Vox 1000 | AMPD Ultra PC |
|---|---|---|---|---|---|---|
| H126 | 12.01 | 16.80 | 16.83 | 13.96 | 16.03 | 16.63 |
| H056 | 13.80 | 17.52 | 21.81 | 19.89 | 18.58 | 22.08 |
| H162 | 32.32 | 32.23 | 31.68 | 32.59 | 31.24 | 29.74 |
| H145 | 17.42 | 20.36 | 19.02 | 16.36 | 18.88 | 23.10 |
| **Total ΔE** | **364.51** | **377.63** | **406.08** | **388.39** | **398.79** | **410.46** |

The total Delta E shows that all the amino alcohols tested give better primary cleaning than the conventional neutralizer MEA. The best, which are compounds according to the invention (all other compounds tested being comparative), are AEPD Vox 1000, CORRGUARD EXT and AMPD Ultra PC (Delta E higher than MEA).

**Claims**

1. A laundry detergent composition comprising:

   (a) 4 to 25 wt% of a linear alkyl benzene sulfonate; (b) 25 to 55 wt% nonionic surfactant, (c) 2 to 10 wt% of an amine having formula (I)

   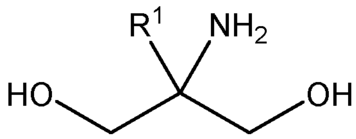
   (I)

   wherein $R^1$ is methyl or ethyl; or formula (II)

   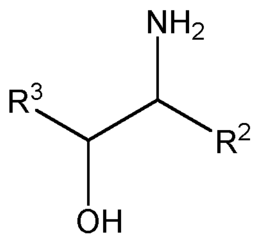
   (II)

   wherein $R^2$ is $C_1$-$C_4$ alkyl and $R^3$ is $C_2$-$C_8$ alkyl; or a combination thereof; and (d) 10 to 35 wt% $C_3$-$C_6$ polyhydroxy compounds.

2. The composition of claim 1 in which the nonionic surfactant is a linear alcohol ethoxylate.

3. The composition of claim 2 comprising from 10 to 30 wt% of an alkyl ether sulfate.

4. The composition of claim 1 in which $R^2$ is methyl or ethyl and $R^3$ is $C_3$-$C_5$ alkyl.

5. The composition of claim 4 comprising from 2.5 to 7 wt% of said amine.

6. The composition of claim 5 comprising 6 to 18 wt% of a linear alkyl benzene sulfonate, 30 to 50 wt% linear alcohol ethoxylate, 15 to 30 wt% $C_3$-$C_6$ polyhydroxy compounds, 12 to 25 wt% alkyl ether sulfate and 2.5 to 7 wt% of said amine.

**Patentansprüche**

1. Eine Waschmittelzusammensetzung, die Folgendes beinhaltet:

   (a) 4 bis 25 Gew.-% eines linearen Alkylbenzolsulfonats; (b) 25 bis 55 Gew.-% nichtionisches Tensid, (c) 2 bis 10 Gew.-% eines Amins mit Formel (I)

   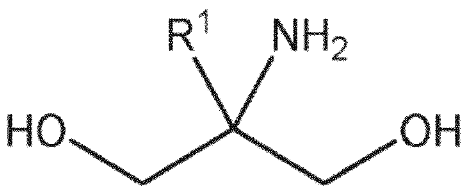

   (I)

   wobei $R^1$ Methyl oder Ethyl ist; oder Formel (II)

   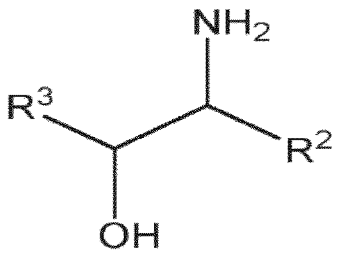

   (II)

   wobei $R^2$ $C_1$-$C_4$-Alkyl ist und $R^3$ $C_2$-$C_8$-Alkyl ist; oder eine Kombination davon; und (d) 10 bis 35 Gew.-% $C_3$-$C_6$-Polyhydroxyverbindungen.

2. Zusammensetzung gemäß Anspruch 1, wobei das nichtionische Tensid ein lineares Alkoholethoxylat ist.

3. Zusammensetzung gemäß Anspruch 2, die zu 10 bis 30 Gew.-% eines Alkylethersulfats beinhaltet.

4. Zusammensetzung gemäß Anspruch 1, wobei $R^2$ Methyl oder Ethyl ist und $R^3$ $C_3$-$C_5$-Alkyl ist.

5. Zusammensetzung gemäß Anspruch 4, die zu 2,5 bis 7 Gew.-% des Amins beinhaltet.

6. Zusammensetzung gemäß Anspruch 5, die 6 bis 18 Gew.-% eines linearen Alkylbenzolsulfonats, 30 bis 50 Gew.-% lineares Alkoholethoxylat, 15 bis 30 Gew.-% $C_3$-$C_6$-Polyhydroxyverbindungen, 12 bis 25 Gew.-% Alkylethersulfat und 2,5 bis 7 Gew.-% des Amins beinhaltet.

**Revendications**

1. Une composition de détergent pour le lavage du linge comprenant :

   (a) de 4 à 25 % en poids d'un alkylbenzènesulfonate linéaire ; (b) de 25 à 55 % en poids de tensioactif non ionique, (c) de 2 à 10 % en poids d'une amine ayant la formule (I)

   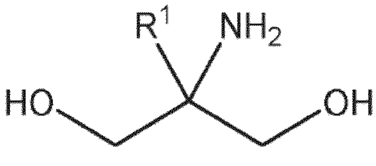

   (I)

   dans laquelle $R^1$ est un méthyle ou un éthyle ; ou la formule (II)

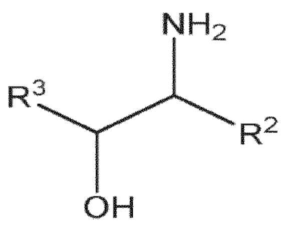

(II)

dans laquelle $R^2$ est un alkyle en $C_1$ à $C_4$ et $R^3$ est un alkyle en $C_2$ à $C_8$ ; ou une combinaison de ceux-ci ; et (d) de 10 à 35 % en poids de composés polyhydroxylés en $C_3$ à $C_6$.

**2.** La composition de la revendication 1 où le tensioactif non ionique est un éthoxylate d'alcool linéaire.

**3.** La composition de la revendication 2 comprenant de 10 à 30 % en poids d'un alkyl éther sulfate.

**4.** La composition de la revendication 1 où $R^2$ est un méthyle ou un éthyle et $R^3$ est un alkyle en $C_3$ à $C_5$.

**5.** La composition de la revendication 4 comprenant de 2,5 à 7 % en poids de ladite amine.

**6.** La composition de la revendication 5 comprenant de 6 à 18 % en poids d'un alkylbenzènesulfonate linéaire, de 30 à 50 % en poids d'éthoxylate d'alcool linéaire, de 15 à 30 % en poids de composés polyhydroxylés en $C_3$ à $C_6$, de 12 à 25 % en poids d'alkyl éther sulfate et de 2,5 à 7 % en poids de ladite amine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5035838 A
- US 4079078 A
- WO 2009125335 A2
- WO 2012067962 A